# EUROPEAN PATENT SPECIFICATION

(11) **EP 2 010 113 B1**
(45) Date of publication and mention of the grant of the patent: **25.07.2012**
(21) Application number: 07732488.7
(22) Date of filing: 20.04.2007
(51) Int. Cl.: A61F 5/058, A61G 7/00, B65D 81/00

(54) **SUPPORT DEVICE**
UNTERSTÜTZUNGSVORRICHTUNG
DISPOSITIF DE SUPPORT

(30) Priority: 21.04.2006 GB 0607955
(43) Date of publication of application: 07.01.2009
(73) Proprietor: Royal National Hospital For Rheumatic Diseases, Upper Borough Walls Bath Bath and North East Somerset BA1 1RL (GB)
(72) Inventor: DOEL, James, North Devon EX33 2EW (GB); BLAKE, David, Bath BA1 2EW (GB); HARRIS, Nigel, Trowbridge BA14 9BG (GB); BRYDEN, Brian, Coventry CV4 8UW (GB)
(74) Representative: Hocking, Adrian Niall
(86) International application number: PCT/GB2007/001448
(87) International publication number: WO 2007/125281

(56) References cited:
- FR-A- 2 045 451
- FR-A1- 2 638 965
- FR-A1- 2 870 318
- US-A- 4 205 667
- US-A- 5 009 318
- US-A- 5 240 135

## Description

The present invention relates to body part support devices.

### Background of the Invention

Many objects requiring transport benefit from immobilisation in a secure manner in order to reduce the chances of damage occurring to the object concerned. Existing forms of support device include the use of polystyrene, or other foam material, blocks, and the use of airbags. However, such devices have limitations, particularly when irregularly shaped objects are considered. Airbags are able to conform to the shape of the object, but are not able to carry tensile loads, and are, therefore, unsuitable for many applications.

In particular, immobilisation of patients having suspected spinal injuries is not served well by existing support devices.

The aim of spinal immobilisation is to stabilise the spine of a patient by restricting mobility, and in so doing prevent exacerbation of spinal cord injury during extrication, resuscitation, transportation, and evaluation of such a trauma patient with suspected spinal instability.

The most widely practiced method of cervical immobilisation is the use of a flat cervical collar. Such collars are essentially flat sections which are wrapped around the neck of a patient to form a tubular support section between the patient's head and neck. The flat sections are typically pre-shaped so as to follow generally the shape of the shoulders and head of the patient.

However, there are a number of problems associated with existing designs of flat packed collar.

Typically, the supporting edges are thin, for example, less than 5mm thick. This means there can be high pressure at the support interface. Medical problems associated with long-term exposure to high pressures are well documented. However, of greater concern to the majority of collar wearers (who have not sustained a spinal injury but are being immobilised as a precautionary measure), is the increase of intracranial pressure which can be detrimental in the prognosis of head and brain injuries.

The existing designs are available only in a limited range of fixed sizes. This means that the fit (that is, the interface at the supporting edges) of the collar and, therefore, the level of immobilisation provided are rarely optimised. Typically three sizes are provided to cover one unit of the population (infant to adult). However, despite there being three sizes available, the current range does not accommodate all patient variations to an acceptable level. Variations include neck sizes (circumferences, heights and postures), head shapes and chin profiles.

The sizes offered by existing designs of collar are determined on the basis of neck height. Variations in neck circumference are accommodated in present designs by an adjustable fastener, which increases the size of the collar or support tube. One problem with this design of support device is that varying the circumference of the collar, or support tube, causes misalignment of the orientation of the pre-formed supporting edges.

When conforming the flat packed collars to the patient it is being done against the shape memory of the material from which the collar is made. This makes the application process unnecessarily awkward and increases the chance of potentially dangerous patient disturbances.

In addition, existing collars are uncomfortable, because the patient is fitted to the collar as opposed to the collar being fitted to the patient. The support mechanism is rigid, fixed in shape and geometry and, therefore, unable to adapt to the shape and form of the patient. Patients often describe the sensation of the collar digging in certain areas and observations of patients have recognised a phenomenon nicknamed the "fidget factor"; fidgeting within the collar to relieve or alter interface pressures and their positions. This fidgeting may exacerbate any existing injuries.

A body part support device as defined in the preamble of claim 1 is disclosed in FR-A-2 045 451.

Accordingly, it is desirable to provide a body part support device which overcomes the disadvantages of the previously considered devices, and which provides additional benefits.

### Summary of the Present Invention

According to one aspect of the present invention, there is provided a body part support device as defined in claim 1.

In one example, the filler material is provided by a plurality of spheres. The spheres may be of substantially solid polystyrene, or may be provided by hollow polystyrene balls.

The means for enabling inflation of the second flexible bag may enable such inflation using pressurised gas. Alternatively, the means for enabling inflation of the second flexible bag may enable such inflation using pressurised fluid. Alternatively, the means for enabling inflation of the second flexible bag may enable such inflation using foam.

According to another aspect of the present invention, there is provided a patient support device comprising at least one such support device.
Such a patient support device may comprise a first part including a first such support device, and a second part including a second such support device.

The first and second parts may be releasably engageable with one another, or may be flexibly engaged with one another.

### Brief Description of the Drawings

Figure 1 is a cross sectional view of a support device embodying the present invention;
Figure 2 is a cross sectional view of the device of Figure 1 in use;
Figure 3 is a cross sectional view of another embodiment of the present invention;
Figure 4 is a plan cross sectional view of a neck restraint incorporating support devices embodying the present invention; and
Figure 5 is a cross sectional view of a further embodiment of the present invention.

### Detailed Description of the Preferred Embodiments

The principles of the present invention will now described with reference to a cervical support collar for immobilising a patient having suspected spinal injury.

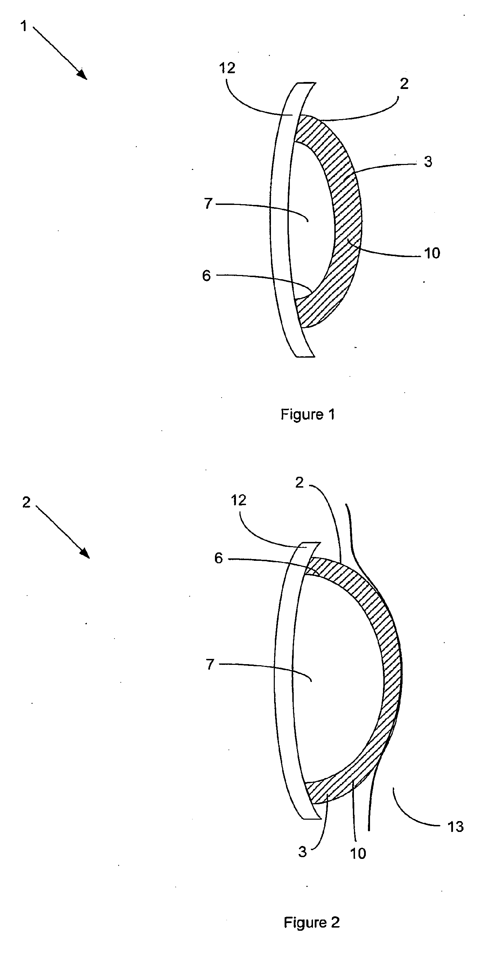

Figure 1 is a cross sectional view of a support device 1 embodying the present invention. The support device 1 comprises a first flexible bag 2 which defines a first enclosed volume 3 therein. A second flexible bag 6 is located within the first enclosed volume 3 within the first flexible bag 2, and defines a second enclosed volume 7. A filler material 10 is provided inside the first enclosed volume 3 and outside of the second enclosed volume. That is, the filler material is provided between the first and second bag 2 and 6. In the embodiment shown in Figure 1, a protective shell 12 supports the first and second bags and filler material. In the unused condition as shown in Figure 1, air in the first and second bags 2 and 6 contain air at substantially atmospheric pressure, such that the first and second bags 2 and 6, and the filler material 10, are substantially flexible.

The first and second bags 2 and 6 are preferably of a tear resistant flexible membrane approximately 0.25mm thick, and may be formed using laser, or radio frequency (RF), welding techniques. Preferably, the protective shell is of a semi-rigid polymer material and is preferably approximately 2mm in thickness.

Figure 2 illustrates the device of Figure 1 in use supporting an object 13. It will be appreciated that the profile of the object in Figure 2 resembles that of a patient's neck. It is to be noted that this application is merely exemplary, and that the support device may be used to support any object or part of a patient that requires support or immobilisation.

In order to support the object 13, the second bag 6 is inflated. Such inflation can be achieved using a pressurised gas supply, preferably air. Alternatively, a liquid, suspension, emulsion or gel could be used to increase the volume of the second bag 6. Inflating the second bag 6 causes the support device 1 to conform to the shape of the object 13. The first bag 2 and the filler material 10 contained therein are thereby also formed to the shape of the object 13.

After the second bag 6 has been inflated to a desired pressure (for example, approximately 1 to 32 psi, approximately 6.8kPa to 220kPa), the first bag is evacuated in order to create at least a partial vacuum in the first enclosed volume 3. Creating such a partial vacuum causes the filler material 10 to be held firmly in place in the conformed shape by the first and second bags 2 and 6. In such a configuration, the support device holds the object 13 substantially rigidly. The evacuated first bag 2 provides the rigidity for the support device 1, and the second bag 6 provides additional support and the conformity of shape.

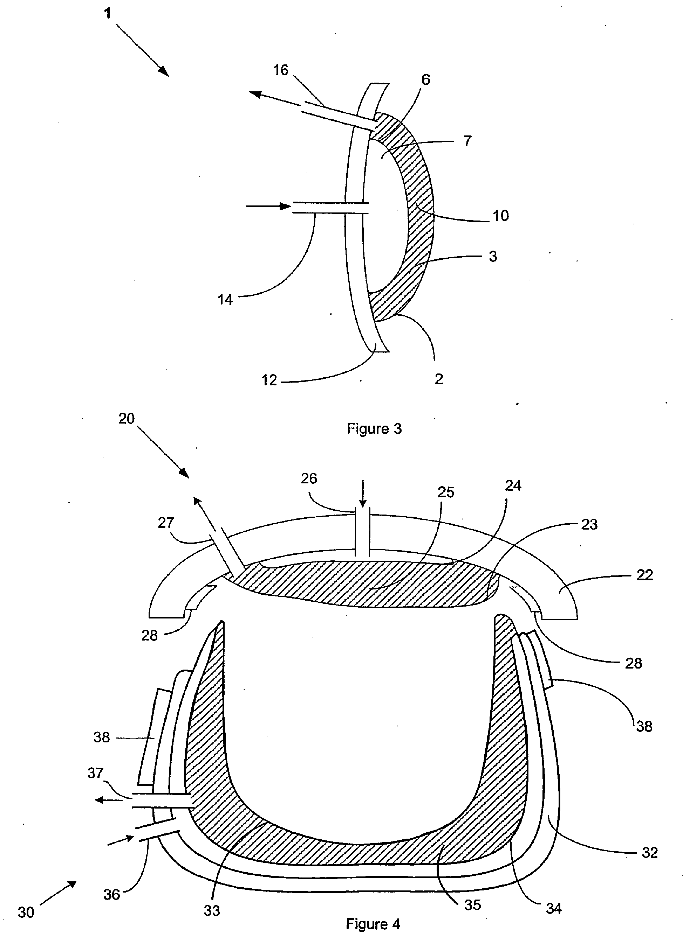

The inflation of the first bag 2 and the evacuation of the second bag 6 can be performed by any appropriate means. Figure 3 illustrates a cross sectional view of another support device that embodies the present invention. The support device 1 of Figure 3 includes an inflation valve 14, and an evacuation valve 16. The inflation valve is provided in order to enable the second bag 6 to be inflated. The evacuation valve 16 is provided in order to enable the first bag 2 to be evacuated. The valves 14 and 16 are preferably provided by non-return valves that can be manually opened when the support device is to be removed.

Figure 4 is a plan cross sectional view of one exemplary neck support collar incorporating the "dual bag" concept of the present invention. In this example, the neck support is provided in two parts: a front part 20 and a rear part 30. The front part 20 comprises a front protector shell 22, first and second flexible bags 23 and 24, and a filler material 25 provided between the first and second bags 23 and 24. An inflation valve 26 is provided in order to allow inflation of the second bag 24, and an evacuation valve 27 is provided in order to enable evacuation of the first bag 23. The first and second bags 23 and 24 of the front part will be referred to as front vacuum bag 23 and front inflation bag 24 for the sake of clarity.

It will be readily appreciated that a support device embodying the present invention may be provided by any number of dual bag elements.

The rear part 30 of the neck support collar comprises a rear protector shell 32 which carries a first bag 33, a second bag 34 and filler material 35 located between the first and second bags 33 and 34. An inflation valve 36 is provided to enable inflation of the second bag 33, and an evacuation valve 37 is provided in order to enable the second bag 34 to be evacuated.

The front and rear protector shells 22 and 32 are preferably preformed in broadly curved shapes for fitting around a patient's neck. The protector shells 22 and 32 may be provided in a flat configuration and bent into shape when used. In one example, the inflation of the second bag causes the protector shells to curve into an appropriate shape.

The front and rear parts 20 and 30 of the neck support device shown in Figure 4 are held together using corresponding fasteners 28 and 38. Preferably, these fasteners are provided by a hook and loop fastener device but could be provided by any appropriate means, such as, for example, a ratchet or other sliding mechanism.

In order to support the head and neck of a patient, the rear part 30 is slid in behind the neck, and the front part 20 is then attached to the rear part 30 using the attachment fasteners 28 and 38. The rear inflation bag 34 is then inflated. After inflation, the rear vacuum bag 33 is evacuated. The inflation and evacuation procedure is carried out for the front inflation bag 24 and front vacuum bag 23 respectively.

The multi-part design of support device enables the device to provide support even if one of the parts is removed, for example to enable to a patient's airway.

In this way, a rigid support device can be provided around the neck of the patient, both to the front and to the rear of the patient. The device is straightforward to fit to the patient, and provides substantially increased immobilisation over previously considered designs. This immobilisation is provided because the inflation bags conform the support device to the exact shape of the patient's neck, and the filler material and vacuum bag provide a substantially rigid structure conformed to that shape.

The filler material may be provided by a large number of small (approximately 2mm in diameter) polystyrene beads. Such beads could be solid or hollow.

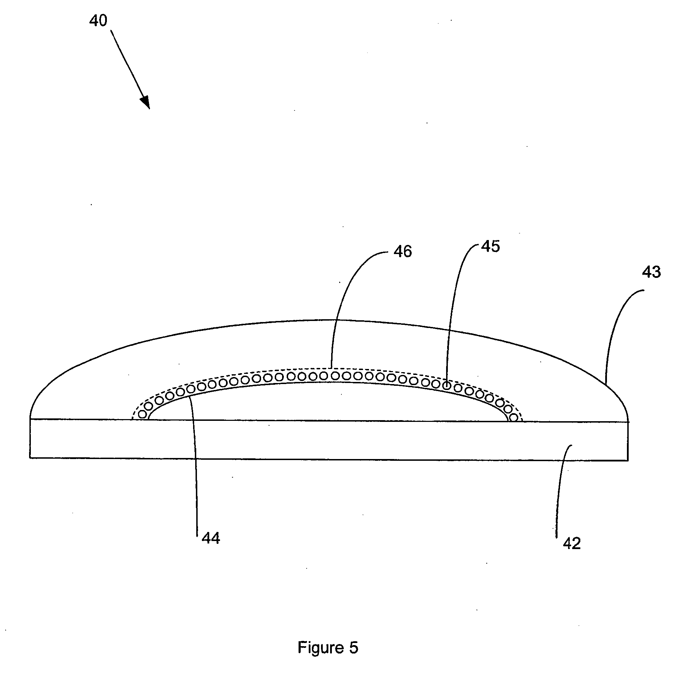

One possible disadvantage of the use of polystyrene beads is that the beads may not be evenly distributed throughout the first bag. Accordingly, Figure 5 illustrates a further embodiment of the present invention which aims to overcome such a disadvantage. The embodiment of Figure 5 comprises a protector shell 42 which carries a vacuum bag 43, and an inflation bag 44. Filler material in the form of polystyrene beads 45 is provided, as airbag and the vacuum bag. In addition, the embodiment of Figure 5 comprises a mesh of flexible material 46 which serves to retain the filler material substantially evenly distributed across the face of the airbag 43. This flexible mesh thereby enables the support device to be used even if the device has been stored in a manner which would otherwise lead to the uneven distribution of the filler material. Subdividing the first bag into compartments could also provide the function of the mesh. Such compartments could be individually evacuated, or could feature valves therebetween to enable all of the compartments to be evacuated during the same evacuation stage.

Although the present invention has been described primarily in connection with neck support devices, it will be readily appreciated that the principles of the invention, namely the use of an inflation bag in combination with a vacuum bag and filler material, can be applied to supporting any part of a patient. For example, an appropriately sized device could be used to immobilise fractured limbs and joints.

A particular advantage of a support device embodying the present invention is that a single device can be used for a range of patient sizes.

## Claims

1. A body part support device (1) comprising:
a first flexible bag (2, 23, 33, 43) which defines a first volume (3) and an inner body-part contact surface;
a second flexible bag (6, 24, 34, 44) which defines a second volume (7);
a filler material (10, 25, 35, 45) which is held in the first bag (2, 23, 33, 43) so as to be external of the second bag (6, 24, 34, 44);
means for enabling inflation of the second flexible bag (6, 24, 34, 44); and
means for enabling evacuation of the first flexible bag (2, 23, 33, 43),
**characterised by** a protective outer shell (12, 22, 32, 42), the first and second flexible bags being supported by the protective outer shell and the second flexible bag extending between the protective outer shell and the first volume.

2. A body part support device (1) as claimed in claim 1, further comprising a mesh of flexible material (46) for substantially retaining the filler material (45) so as to be substantially evenly distributed in the first bag (43).

3. A body part support device (1) as claimed in claim 1, further comprising at least one divider for subdividing the first bag (43) into compartments so as to substantially evenly distribute the filler material (45) across the first bag (43).

4. A body part support device (1) as claimed in claim 3, wherein each said compartment includes a valve therebetween for evacuation.

5. A body part support device (1) as claimed in claim 3, wherein the compartments are individually evacuatable.

6. A body part support device (1) as claimed in any one of the preceding claims, wherein the second flexible bag (34) overlaps the protective outer shell (32).

7. A body part support device (1) as claimed in any one of the preceding claims, wherein the filler material (10, 25, 35, 45) is provided by a plurality of spheres.

8. A body part support device (1) as claimed in claim 6, wherein the spheres are of substantially solid polystyrene or hollow polystyrene balls.

9. A body part support device (1) as claimed in any one of the preceding claims, wherein the means for enabling inflation of the second flexible bag (6, 24, 34, 44) enable such inflation using pressurised gas or pressurised fluid or foam.

10. A patient support device (1) comprising at least one body part support device (1) as claimed in any one of the preceding claims.

11. A patient support device (1) comprising a first part (20) including a body part support device as claimed in anyone of the preceding claims, and a second part (30) including a body part support device as claimed in anyone of the preceding claims.

12. A patient support device (1) as claimed in claim 10, wherein the first and second parts (20, 30) are releasably engageable with one another.

13. A patient support device (1) as claimed in claim 11, wherein the first part (20) is flexibly engaged with the second part (30).

## Patentansprüche

1. Unterstützungsvorrichtung (1) für Körperteile, aufweisend:
einen ersten flexiblen Beutel (2, 23, 33, 43), der ein erstes Volumen (3) und eine innere Kontaktfläche für einen Körperteil definiert;
einen zweiten flexiblen Beutel (6, 24, 34, 44), der ein zweites Volumen (7) definiert;
ein Füllmaterial (10, 25, 35, 45), das im ersten Beutel (2, 23, 33, 43) aufbewahrt wird, sodass es außerhalb des zweiten Beutels (6, 24, 34, 44) ist;
Mittel zum Ermöglichen des Aufblasens des zweiten flexiblen Beutels (6, 24, 34, 44); und
Mittel zum Ermöglichen der Evakuierung des ersten flexiblen Beutels (2, 23, 33, 43),
**gekennzeichnet durch** ein schützendes Außengehäuse (12, 22, 32, 42), wobei der erste und der zweite flexible Beutel vom schützenden Außengehäuse gestützt werden und sich der zweite flexible Beutel zwischen dem schützenden Außengehäuse und dem ersten Volumen erstreckt.

2. Unterstützungsvorrichtung (1) für Körperteile nach Anspruch 1, des Weiteren aufweisend ein Gewebe aus einem flexiblen Material (46), im Wesentlichen für das Beinhalten des Füllmaterials (45), sodass es im Wesentlichen gleichmäßig im ersten Beutel (43) verteilt ist.

3. Unterstützungsvorrichtung (1) für Körperteile nach Anspruch 1, des Weiteren aufweisend zumindest einen Teiler zum Unterteilen des ersten Beutels (43) in Abteile, um das Füllmaterial (45) im Wesentlichen gleichmäßig im ersten Beutel (43) zu verteilen.

4. Unterstützungsvorrichtung (1) für Körperteile nach Anspruch 3, wobei jedes Abteil ein Ventil dazwischen für die Evakuierung enthält.

5. Unterstützungsvorrichtung (1) für Körperteile nach Anspruch 3, wobei die Abteile individuell evakuierbar sind.

6. Unterstützungsvorrichtung (1) für Körperteile nach einem der vorhergehenden Ansprüche, wobei der zweite flexible Beutel (34) das schützende Außengehäuse (32) überlappt.

7. Unterstützungsvorrichtung (1) für Körperteile nach einem der vorhergehenden Ansprüche, wobei das Füllmaterial (10, 25, 35, 45) durch eine Mehrzahl von Kugeln bereitgestellt wird.

8. Unterstützungsvorrichtung (1) für Körperteile nach Anspruch 6, wobei die Kugeln aus im Wesentlichen massivem Polystyrol oder hohlen Polystyrolkugeln bestehen.

9. Unterstützungsvorrichtung (1) für Körperteile nach einem der vorhergehenden Ansprüche, wobei die Mittel zum Ermöglichen des Aufblasens des zweiten flexiblen Beutels (6, 24, 34, 44) ein solches Aufblasen durch Druckgas, Druckfluid oder Schaum ermöglichen.

10. Patientenunterstützungsvorrichtung (1), aufweisend zumindest eine Unterstützungsvorrichtung (1) für Körperteile nach einem der vorhergehenden Ansprüche.

11. Patientenunterstützungsvorrichtung (1), aufweisend einen ersten Teil (20), der eine Unterstützungsvorrichtung für Körperteile nach einem der vorhergehenden Ansprüche enthält, und einen zweiten Teil (30), der eine Unterstützungsvorrichtung für Körperteile nach einem der vorhergehenden Ansprüche enthält.

12. Patientenunterstützungsvorrichtung (1) nach Anspruch 10, wobei der erste und der zweite Teil (20, 30) lösbar miteinander in Eingriff gehen können.

13. Patientenunterstützungsvorrichtung (1) nach Anspruch 11, wobei der erste Teil (20) flexibel mit dem zweiten Teil (30) in Eingriff steht.

## Revendications

1. Dispositif (1) de support de partie de corps comprenant :
une première poche souple (2, 23, 33, 43) qui définit un premier volume (3) et une surface interne de contact avec une partie de corps ;
une seconde poche souple (6, 24, 34, 44) qui définit un second volume (7) ;
une matière de remplissage (10, 25, 35, 45) qui est contenue dans la première poche (2, 23, 33, 43) de façon à être externe à la seconde poche (6, 24, 34, 44) ;
des moyens pour permettre le gonflage de la seconde poche souple (6, 24, 34, 44) ; et
des moyens pour permettre l'évacuation de la première poche souple (2, 23, 33, 43),
**caractérisé par** une enveloppe externe de protection (12, 22, 32, 42), les première et seconde poches souples étant supportées par l'enveloppe externe de protection et la seconde poche souple s'étendant entre l'enveloppe externe de protection et le premier volume.

2. Dispositif (1) de support de partie de corps selon la revendication 1, comprenant en outre un treillis de matière souple (46) pour retenir sensiblement la matière de remplissage (45) de telle sorte qu'elle est répartie sensiblement uniformément dans la première poche (43).

3. Dispositif (1) de support de partie de corps selon la revendication 1, comprenant en outre au moins un diviseur pour subdiviser la première poche (43) en compartiments de façon à répartir sensiblement uniformément la matière de remplissage (45) à travers la première poche (43).

4. Dispositif (1) de support de partie de corps selon la revendication 3, dans lequel chaque compartiment précité comprend entre eux une soupape pour l'évacuation.

5. Dispositif (1) de support de partie de corps selon la revendication 3, dans lequel les compartiments sont aptes à être évacués individuellement.

6. Dispositif (1) de support de partie de corps selon l'une quelconque des revendications précédentes, dans lequel la seconde poche souple (34) chevauche l'enveloppe externe de protection (32).

7. Dispositif (1) de support de partie de corps selon l'une quelconque des revendications précédentes, dans lequel la matière de remplissage (10, 25, 35, 45) est fournie par une pluralité de sphères.

8. Dispositif (1) de support de partie de corps selon la revendication 6, dans lequel les sphères sont en billes de polystyrène sensiblement solides ou en billes de polystyrène creuses.

9. Dispositif (1) de support de partie de corps selon l'une quelconque des revendications précédentes, dans lequel les moyens pour permettre le gonflage de la seconde poche souple (6, 24, 34, 44) permettent un tel gonflage à l'aide d'un gaz sous pression ou d'un fluide sous pression ou d'une mousse.

10. Dispositif (1) de support de patient comprenant au moins un dispositif (1) de support de partie de corps tel que défini à l'une quelconque des revendications précédentes.

11. Dispositif (1) de support de patient comprenant une première partie (20) comprenant un dispositif de support de partie de corps tel que défini à l'une quelconque des revendications précédentes, et une seconde partie (30) comprenant un dispositif de support de partie de corps tel que défini à l'une quelconque des revendications précédentes.

12. Dispositif (1) de support de patient selon la revendication 10, dans lequel les première et seconde parties (20, 30) sont aptes à venir en prise de manière libérable l'une avec l'autre.

13. Dispositif (1) de support de patient selon la revendication 11, dans lequel la première partie (20) est en prise de manière souple avec la seconde partie (30).
